# EUROPEAN PATENT SPECIFICATION

(11) **EP 2 391 394 B1**
(45) Date of publication and mention of the grant of the patent: **17.12.2014**
(21) Application number: 10700886.4
(22) Date of filing: 08.01.2010
(51) Int. Cl.: A61L 15/32, A61L 15/44, A61L 26/00

(54) **GEL LAYER**
GELSCHICHT
COUCHE DE GEL

(30) Priority: 09.01.2009 GB 0900326
(43) Date of publication of application: 07.12.2011
(73) Proprietor: MBP Medical Biomaterial Products GmbH, 19306 Neustadt-Glewe (DE)
(72) Inventor: Barker, Stephen George Edward, Shirley, Surrey CR0 8UJ (GB); Behjati, Barat, 19304 neustadt-Glewe (DE)
(74) Representative: Prinz & Partner mbB
(86) International application number: PCT/GB2010/000033
(87) International publication number: WO 2010/079342

(56) References cited:
- WO-A1-03/101501
- GB-A- 1 205 609
- GB-A- 1 298 223
- MAEDA M ET AL: "Sustained release of human growth hormone (hGH) from collagen film and evaluation of effect on wound healing in db/db mice" JOURNAL OF CONTROLLED RELEASE, ELSEVIER, AMSTERDAM, NL LNKD- DOI:10.1016/S0168-3659(01)00512-0, vol. 77, no. 3, 13 December 2001 (2001-12-13), pages 261-272, XP004328579 ISSN: 0168-3659

## Description

### Field of the invention

The invention relates to a gel layer suitable for use as a skin dressing, and to a wound dressing comprising the gel layer. It is also concerned with a cosmetic method for improving the appearance of skin, and a gel layer for topical use in treating a wound.

### Background to the invention

Collagen is a fibrous protein that is a major structural component of connective tissue (e.g. ligaments, tendons, cartilage and skin) in humans and animals. In medical applications, collagen is commonly used in cosmetic facial treatments to augment lips or to fill in wrinkles. It has also been used as a healing aid in the surgical treatment of burns. Collagen is known to assist or promote wound healing by stimulating growth factors. It can also provide a haemostatic effect.

Collagen in the form of a sponge (known as a collagen sponge or a collagen haemostat) has been used in surgery to control bleeding, see US 3,157,524. Collagen sponge is an absorbent material and is generally used as a compress. It is prepared by lyophilising an aqueous dispersion of collagen fibres to dryness. The resulting sponge has an open pore structure and generally has a density from 0.015 g.cm⁻³ to 0.060 g.cm⁻³.

US 2006/0159731 describes a multi-layer article for use in healing wounds. The article has a first layer for contacting a wound that comprises non or partially cross-linked collagen, and a second layer that comprises highly cross-linked collagen. The multi-layer structure of the article provides a dressing that has a layer for promoting cell-growth and a layer for the protection of injured tissue. A multi-layered collagen sponge is exemplified in US 2006/0159731. The flexibility of this article may be improved by treating the sponge (post-lyophilisation) with a plasticizer, such as glycerol.

M. Maeda et al (Journal of Controlled Release, 77(3), (2001), 261-271) describes collagen films containing human growth hormone.

WO 03/101501 describes a mutli-layer collagen article useful for wound healing comprising at least two layers, wherein at least one layer for facing the wound side comprises an effective amount of non or partially cross-linked collagen, and at least one layer comprises an effective amount of highly cross-linked collagen matrices.

GB 1298223 describes a method of making an absorbent collagen network comprising forming a homogenised collagen slurry having a dry collagen content of 0.3 to 3%, having a pH below 5.5 and including a non-collagenous material that will promote cross-linking of the slurry while frozen, the slurry being formed by a process comprising forming a suspension of comminuted untanned collagen tissue and homogenising this, freezing the slurry at -2 to -40 °C, maintaining it at this temperature for 12 hours to 30 days, thawing the frozen slurry and then removing by squeezing most of the water that was in the frozen slurry.

### Summary of the invention

It has been found that a gel layer comprising a homogeneous mixture of glycerol and collagen has a number of advantageous properties. The gel layer of the invention is transparent, pliable, conformable to a skin surface, moisture absorbent, tacky, and feels "cool" to touch. Importantly, it also retains its structural integrity after prolonged immersion in water. The properties of the gel layer are particularly suited for it to be used as a skin dressing.

The present invention therefore provides a gel layer suitable for use as a skin dressing, according to claim 1.

The invention further provides a gel layer suitable for use as a skin dressing, wherein said layer comprises a homogeneous mixture of glycerol and non-crosslinked atelopeptide collagen, according to claim 11.

The invention also provides a wound dressing suitable for topical use, comprising a gel layer of the invention, and a retaining layer for securing the dressing to skin or to a wound area.

It also provides a cosmetic method of improving the appearance of skin, which comprises applying a gel layer of the invention to the skin of a human or animal patient.

In a further aspect, the invention provides a gel layer for topical use in treating a wound.

### Brief description of drawings

Figure 1 is a flow chart that shows the steps involved in the production of a gel layer in an embodiment where collagen is obtained from porcine skin. The collagen used to prepare the gel layer could be collagen from another source, such as bovine collagen. Figure 2 is a photograph of a typical collagen sponge.
Figure 3 is a photograph of a gel layer of the invention. As shown, some bubbles may be formed within the gel layer.
Figure 4 is a photograph of a gel layer of the invention, intact after it has been soaked in water for at least 12 hours.

### Detailed description of the invention

The gel layer of the invention is typically transparent. The transparency of the gel layer is advantageous because it permits inspection of a wound when covered by the gel layer.

Typically, the gel layer of the invention is pliable. It is, preferably, conformable to the anatomy to which it is to be applied. The gel layer is able to conform fully to a skin surface or wound area.

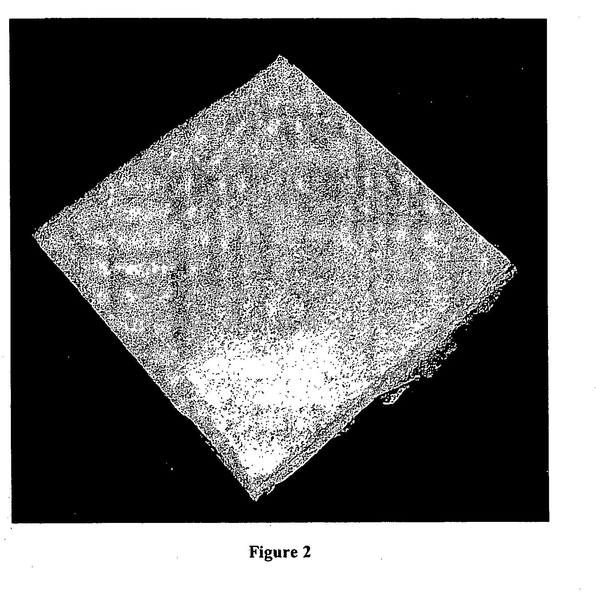

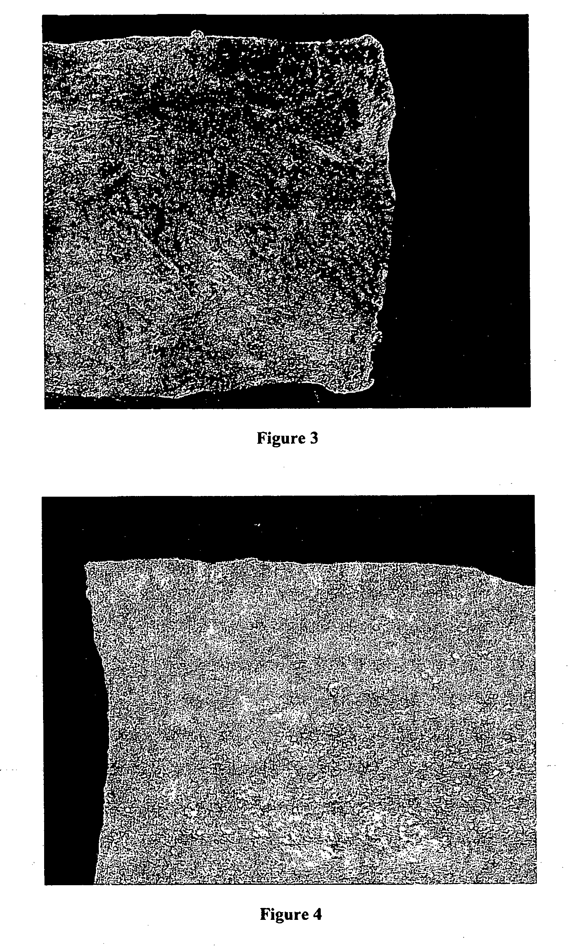

Unlike conventional collagen sponges, the gel layer of the invention is substantially non-porous. Thus, although the gel layer of the invention will absorb water, it does not have an open-pore structure allowing for rapid ingress of fluid. The non-porous nature of the gel layer, as compared to a collagen sponge as shown in Figure 2, is evident from Figure 3. Typically, the gel layer of the invention is moisture absorbent. The gel layer can typically absorb liquids, such as moisture or exudates from wounds or skin conditions, by diffusion. This is particularly beneficial in for example, burns, which may weep significant fluid volumes.

Typically, the gel layer of the invention retains its structural integrity when soaked in water. Thus, although the gel layer may swell in the presence of moisture it typically does not dissolve. Thus, the gel layer of the invention typically will not dissolve into a wound after application. A gel layer that has been soaked in water for at least 12 hours is shown in Figure 4.

Preferably, the thickness of the gel layer of the invention increases by from 50% to 300%, more preferably from 50% to 200%, particularly 50% to 100% following immersion in water for 12 hours at room temperature. The gel layer also typically became opaque (see Figure 4).

Typically, the gel layer of the invention provides a cooling sensation when applied to skin or a wound. This sensation is surprising because the gel layer on initial placement does not contain any significant quantity of water. This property is particularly beneficial for bums, where the cooling sensation is soothing to the patient.

Typically, the gel layer is tacky. Thus, the gel layer may cling or stick lightly to itself, to a wound area, or to skin. The adherency of the gel layer is generally from 5 to 200 g, preferably from 20 to 60 g. Typically, said adherency of the gel layer is measured by sticking 25 mm wide strips of the gel layer onto dry skin, and allowing a weight attached to one end of the strip to draw the gel layer gravitationally from the skin at an angle of 160° thereto. The weight that draws the dressing from the skin at a speed of 1 mm/s is a measurement of the adherency of the gel layer.

As is evident from the above figures, the gel sheet of the invention is not, typically, strongly adherent. Thus, it is removable from skin without pulling the skin away from the body. In clinical terms, the gel layer of the invention would be classified as a "non-adherent" wound dressing.

Typically, the gel layer of the invention is elastically deformable. Thus, the gel layer is typically tear resistant. Upon application of a moderate tensile force (e.g. when the sides of the article are gently pulled apart), the gel stretches and will return to its original shape. The gel layer of the invention is, typically, tear resistant after it has been soaked or immersed in water. Typically, the gel layer of the invention has a Youngs modulus of from 5 kPa to 1000 kPa, preferably from 50 kPa to 500 kPa, and more preferably from 100 kPa to 300 kPa. These values are measured at a temperature of 25 °C.

Generally, the tear resistance of the gel layer after it has been soaked or immersed in water, usually for at least one hour, is greater (typically at least 5 % greater, preferably at least 10 % greater) than the tear resistance of the gel layer before it has been soaked or immersed in water.

The gel layer of the invention is an integral article. Thus, the gel layer cannot be rubbed into the skin like an ointment or a cream.

As used herein, the term "homogeneous mixture", in the context of the gel layer of the invention, refers to a mixture having a substantially uniform distribution of collagen protein molecules and glycerol molecules throughout. Thus, a dispersion of collagen protein aggregates in a glycerol solvent typically would not constitute a homogeneous mixture. Typically, the gel layer of the present invention does not contain collagen aggregates having a primary particle size greater than 10 microns mixed with glycerol.

Typically, the gel layer of the invention is substantially anhydrous. More typically, the amount of water in the gel layer is less than or equal to 1% by weight of the gel layer. It is preferred that the amount of water in the gel layer is less than or equal to 0.5% by weight of the gel layer, more preferably less than or equal to 0.1% by weight of the gel layer and even more preferred the amount of water in the gel layer is less than or equal to 0.01% by weight of the gel layer.

An aspect of the invention relates to a gel layer obtainable by lyophilising an aqueous mixture comprising glycerol and collagen. In general, the aqueous mixture comprising glycerol and collagen is prepared by mixing glycerol with an aqueous dispersion of collagen, to form a homogenous mixture.

Typically, the aqueous mixture comprising glycerol and collagen is lyophilised until substantially anhydrous. Lyophilisation or freeze drying is a well known process of removing water from a substance, such as the aqueous mixture comprising collagen and glycerol, by sublimation.

Normally, lyophilisation of the mixture is performed for at least 15 hours. It is preferred that lyophilisation of the mixture is performed for at least 18 hours, typically for 18 to 20 hours. The lyophilisation time required may vary depending on the freeze drying machine and the amount of the aqueous mixture to be lyophilised.

Generally, the gel layer of the invention comprises glycerol of a reagent grade that is suitable for use in medical applications.

In principle, any type of collagen may be used in the gel layer of the invention. Suitable types of collagen include bovine collagen, fish collagen, porcine collagen, avian collagen (e.g. ostrich collagen), recombinant human collagen or mixtures thereof. Normally, the collagen is a medical grade collagen that is suitable for inclusion in medicinal products, such as wound dressings. It is preferred that the collagen is porcine collagen, bovine collagen, avian collagen or recombinant human collagen. More preferably the collagen is porcine collagen or recombinant human collagen. These types of collagen have better compatibility with human skin and minimise the occurrence of undesirable side effects, such as rejection, irritation and an immune response. Typically, the collagen is porcine collagen.

The gel layer preferably comprises atelopeptide collagen, more particularly non-crosslinked atelopeptide collagen. The reference to "atelopeptide collagen" used herein refers to collagen that has been treated to separate and remove the telopeptides from the collagen. The term "non-crosslinked" refers to collagen that has not been treated with a reagent (generally known as a cross-linking agent) to crosslink it to itself or to another material, such as a synthetic polymer. Crosslinking collagen may strengthen the collagen containing material and the lifetime of the resulting product may be increased. Generally, a wound or skin is dressed by a gel layer of the invention for no more than 48 hours, preferably no more than 24 hours.

In the present invention, it is preferred that an aqueous mixture comprising glycerol and collagen is prepared by mixing glycerol with an aqueous dispersion of collagen. Suitable aqueous dispersions of collagen may be prepared by any conventional method. Such methods are disclosed, for example, in US 3,034,852, US 3,121,049, US 3,530,037 and US 4,424,208. The aqueous dispersion of collagen usually has the appearance of "ready to use" wall-paper paste.

The amount of collagen in the aqueous dispersion is typically from 0.1% to 10% by weight of the dispersion, preferably from 0.3% to 5% by weight of the dispersion, even more preferred is from 0.5% to 2.5% by weight of the dispersion, especially from 0.75% to 1.5% by weight of the dispersion.

The gel layer of the invention may be prepared from an aqueous mixture comprising about 1% to 10% by weight of glycerol and about 90% to 99% by weight of an aqueous collagen dispersion. The total percentage weight of the glycerol and aqueous collagen dispersion in the mixture may have to be adjusted if other ingredients or components are included in the aqueous mixture. In embodiments where other ingredients are included in the aqueous mixture, then the aqueous mixture is composed of at least 50% by weight, preferably 60% by weight, more preferably 75% and especially 90% by weight of a combination of glycerol and the aqueous collagen.

In the present invention, the ratio of glycerol to collagen in the gel layer (or the aqueous mixture) is from 50:1 to 1:1 by weight, more preferably from 25:1 to 3:2 by weight. More preferably the ratio of glycerol to collagen is from 15:1 to 2:1 by weight, especially from 10:1 to 3:1 by weight, and most preferred the ratio of glycerol to collagen is from 15:2 to 4:1 by weight.

In general, the gel layer is free of additives. However, it may be desirable in some instances for to include a preservative in the gel layer. Thus, in an embodiment of the invention the gel layer comprises a preservative.

The gel layer of the invention may be substantially anhydrous and consist of a homogeneous mixture of collagen, glycerol, and optionally a preservative.

The gel layer may provide a means for carrying and delivering a therapeutic agent or other active ingredient for the treatment of a wound area or skin, or for cosmetically improving the appearance of skin. Typically, the therapeutic agent or active ingredient is suitable for topical application.

Thus, the gel layer of the invention may comprise a therapeutic agent that is an antibiotic, an antibacterial agent, an antiviral agent, an antifungal agent, a growth factor, a corticosteroid, a prostaglandin, a cytokine, zinc ions, hyaluronic acid, fibronectin, a proteoglycan, an alginate or a combination thereof.

Suitable antibiotics include gentamicin, penicillin, tetracycline, oxytetracycline, chlorotetracycline, chloramphenicol or combinations of one or more of these antibiotics. The gel layer of the invention preferably comprises gentamicin.

Suitable antibacterial agents include a sulphonamide compound and silver ions (i.e. a silver salt compound). Suitable sulphonamide compounds include celecoxib, mafenide, sulfacetamide, sulfamethoxazole and sulphadiazine. The gel layer may, for example, comprise silver sulphadiazine. The silver sulphadiazine provides the silver ions in the gel layer. It is preferred that the gel layer comprises silver ions.

Suitable growth factors include angiogenic growth factors (particularly for ischemic wounds), such as platelet derived growth factor (PDGF). It is preferred that the gel layer comprises PDGF. A gel layer of the invention which comprises PDGF is particularly useful for the treatment of diabetic foot ulcers.

Suitable prostaglandins include iloprost and prostaglandin E₁ (PGE₁).

In one embodiment, the gel layer may comprise nanoparticles, in particular metallic nanoparticles such as those comprising gold, silver, copper or an alloy thereof, and photosensitiser(s). The combination of nanoparticles and photosensitiser(s) may act as an antimicrobial agent, which is capable of killing or inhibiting the growth of microorganisms, such as bacteria, viruses, fungi and prions. The term "nanoparticles" refers to particles having a diameter of from 1 to 100 nm, preferably from 1 to 30 nm. A photosensitiser is a compound that can be excited by light of a specific wavelength. Thus, such a compound may have an absorption band in the ultraviolet, visible or infrared portion of the electromagnetic spectrum and, when the compound absorbs radiation within that band, it generates cytotoxic species, thereby exerting an antimicrobial effect.

The photosensitiser is suitably chosen from porphyrins (e.g. haematoporphyrin derivatives, deuteroporphyrin), phthalocyanines (e.g. zinc, silicon and aluminium phthalocyanines), chlorins (e.g. tin chlorin e6, poly-lysine derivatives of tin chlorin e6, m-tetrahydroxyphenyl chlorin, benzoporphyrin derivatives, tin etiopurpurin), bacteriochlorins, phenothiaziniums (e.g. toluidine blue O, methylene blue, dimethylmethylene blue), phenazines (e.g. neutral red), acridines (e.g. acriflavine, proflavin, acridine orange, aminacrine), texaphyrins, cyanines (e.g. merocyanine 540), anthracyclins (e.g. adriamycin and epirubicin), pheophorbides, sapphyrins, fullerene, halogenated xanthenes (e.g. rose bengal), perylenequinonoid pigments (e.g. hypericin, hypocrellin), gilvocarcins, terthiophenes, benzophenanthridines, psoralens and riboflavin. Other possibilities are arianor steel blue, tryptan blue, crystal violet, azure blue cert, azure B chloride, azure 2, azure A chloride, azure B tetrafluoroborate, thionin, azure A eosinate, azure B eosinate, azure mix sicc. and azure II eosinate. Preferred photosensitisers include toluidine blue O, methylene blue, dihaematoporphyrin ester, tin chlorin e6, indocyanine green, rose bengal, methylene violet, erythrosine B and nile blue sulphate.

Typically, the nanoparticles comprise a metal, preferably nanoparticles comprising a main group metal or a transition metal, such as gold, silver or copper or an alloy thereof, particularly silver, silver alloy and/or silver oxide. The nanoparticles may be charge-stabilized or ligand-stabilized.

The nanoparticles and photosensitiser(s) may be separate entities. When the nanoparticles and photosensitiser(s) are separate, then a preferred combination is gold nanoparticles and methylene blue or toluidine blue O as a photosensitiser. Alternatively, the nanoparticles and photosensitiser may be associated, e.g. as a metallic nanoparticle-ligand-photosensitiser conjugate. When the nanoparticles and photosensitiser are associated, then preferably the nanoparticles comprise gold, the ligand comprises tiopronin and the photosensitiser comprises toluidine blue O. Suitable examples of the aforementioned combinations of nanoparticles and photosensitiser(s), both separate and conjugated, may be found in WO 2008/015453.

Generally, the inclusion of a therapeutic agent or other active agent or ingredient mentioned herein should not substantially alter the basic characteristics of the gel layer. In particular, the inclusion of a therapeutic agent or other active agent or ingredient mentioned herein should not substantially alter the transparency of the gel layer.

The gel layer of the invention may comprise an anti-ageing agent, an anti-wrinkle agent, an antioxidant, an anti-scarring agent, an antiseptic anti-acne agent or a combination thereof. A gel layer comprising one or more of these agents is particularly suitable for use in a cosmetic method of the invention.

The invention also provides a gel layer comprising a biological glue, a pH buffer, a drying enhancer or a combination thereof. A gel layer comprising a biological glue, a pH buffer or a drying enhancer is useful in the therapeutic treatment of a wound or for cosmetically improving the appearance of skin.

In one embodiment of the invention, the gel layer is substantially free from chitin, chitosan, chitinamine or an acid salt of chitinamine, and/or is substantially free from a biocompatible polymer, particularly a biocompatible polymer selected from polyethylene glycol (PEG), poly-L-lysine, alginate, chitosan, hyaluronic acid, chondroitin sulfate and mixtures thereof.

The gel layer of the invention may have perforations. The perforations allow a patient's skin to breathe or may facilitate the bulk drainage of wound exudates or moisture from skin.

Generally, the gel layer of the invention is obtainable by placing an aqueous mixture comprising glycerol and collagen into a mould, then lyophilising the mixture until it is substantially anhydrous to form the gel layer. The size of the mould usually determines the physical dimensions of the gel layer. The mould is, for example, made of perspex and has a rectangular in shape with a depth of 3 to 4 mm. A mixture of 25 mL of collagen and glycerol may be used to prepare a gel layer of 5 cm by 5 cm with a thickness of approximately 1.5 mm.

Typically, the gel layer of the invention is generally rectangular or square in shape. The rectangular or square gel sheets generally have a length and width of from 1 cm to 10 cm. The thickness of the gel sheet is typically from 0.5 mm to 2 mm (usually around 1.5 mm).

The therapeutic agent, other active agents or ingredients described above are typically included in the aqueous mixture comprising glycerol and collagen before the step of lyophilising said mixture.

Another aspect of the invention relates to a wound dressing suitable for topical use, comprising a gel layer of the invention, and a retaining layer for securing the dressing to skin or to a wound area. It is preferred that the retaining layer is an outer layer of the dressing having at least one adhesive portion that extends beyond the gel layer. The adhesive portion is preferably a part of the retaining layer that is coated with a suitable medical-grade adhesive. The adhesive portion generally extends beyond, or hangs over, an edge of the gel layer.

The wound dressing of the invention may further comprise a layer of an absorbent material. It is preferred that the layer of absorbent material is in contact with a surface of the gel layer. Typically, the absorbent material is a super-absorbent material, such as a super-absorbent polymer. Super-absorbent materials are well known in the art. The absorbent material may be used to provide a further layer for absorbing moisture, wound exudates or such like, that seep through the gel layer of the dressing. Normally, the layer of absorbent material is sandwiched between the retaining layer and the gel layer.

When the wound dressing of the invention comprises a layer of an absorbent material, it is preferred that the gel layer has perforations.

To prevent the gel layer from absorbing atmospheric moisture before use, the wound dressing may further comprise a peelable cover sheet that is releasably attached to a surface of the gel layer. In use, the cover sheet is removed and separated from the gel layer shortly before application of the dressing. Preferably, the cover sheet has overlapping pull tabs to facilitate its release from the surface of the gel layer.

The dressings of the invention are preferably packaged under sterile conditions and are sealed in a packet or pouches that are impermeable to water. Before packaging, the dressing of the invention may be sterilised by exposure to gamma irradiation, electron beam irradiation, heat sterilisation or treating it with ethylene oxide. A dressing of the invention may be sterilised after it has been packaged by exposing it to gamma irradiation or electron beam irradiation in the packaging.

- Another aspect of the invention relates to a cosmetic method of improving the appearance of skin, which comprises applying a gel layer of the invention to the skin of a human or animal patient. Collagen is included in many anti-ageing creams because it may to reduce the appearance of wrinkles or other signs of skin ageing.

In this embodiment, it is preferred that the gel layer comprises an anti-ageing agent, an anti-wrinkle agent, an antioxidant, an anti-scarring agent, an antiseptic anti-acne agent or a combination thereof. Such ingredients are useful in the cosmetic treatment of skin conditions that include the visible effects of aging, wrinkles, acne, age spots, scars (hypertrophic or keloid) and broken capillaries (telangiectases). More preferably, the gel layer comprises an anti-ageing agent, an anti-wrinkle agent or an antioxidant.

Another aspect of the invention relates to a gel layer of the invention for topical use in treating a wound. A gel layer of the invention may be applied or administered to the human or animal body for the treatment of bums; sores; ulcers (especially diabetic ulcers, leg ulcers and other cavity wounds) or ulcerated areas of skin; open wounds, such as cuts, grazes, puncture wounds, punctures, lacerations and other such traumatic injuries; the skin remaining at a donor site after a skin graft; and to minimise hypertrophic or keloid scarring, particularly scarring that may arise from the treatment of a wound. It is preferred that the gel layer is applied or administered to a burn.

Typically, a dressing or gel layer of the invention may be used as a cannula dressing or a catheter dressing.

A dressing or gel layer of the invention may be used in combination with one or more other dressings known in the art. The dressing or gel layer of the invention may be cut or trimmed into a desired shape or size before application using a scissors or scalpel.

The invention will now be illustrated by the following, non-limiting example.

### Example 1

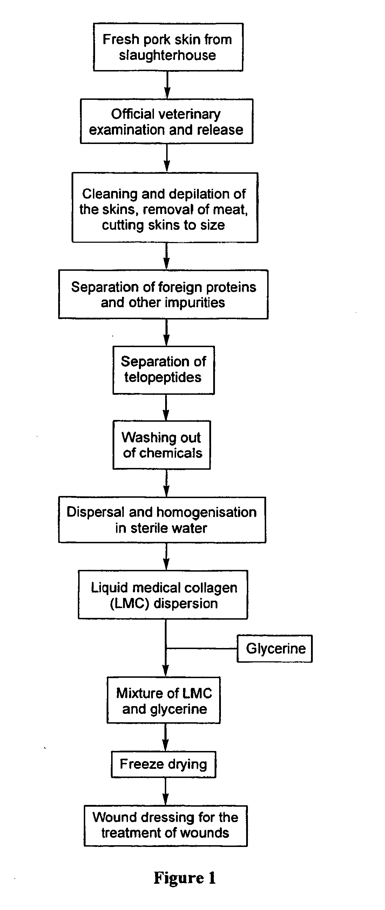

As shown in the flow chart of Figure 1, collagen may be obtained from processed animal hide, such as pig skin. A collagen "slurry" of finely shred, de-cellularised porcine dermis and water (at an approximate mix of 1% collagen to 99% water) was thoroughly mixed. The resulting mixture has the appearance of wallpaper paste. 550 mL of this aqueous dispersion of collagen was thoroughly mixed with 42.7 mL of glycerol, then poured into a series of suitably sized moulds to produce gel sheets of a desired size. The moulds were placed into a vacuum chamber of a freeze dryer, which was then sealed and cooled to -35°C (± 2°C). The moulds were then held at this temperature for 18 hours (± 1 hour) to freeze the water present. The pressure inside the chamber was then lowered to 0.65 mbar by forcing air out of the chamber and the temperature was raised using a heater to -5 °C for 4 hours to start sublimation of the water. The sublimed water is trapped using a freezing coil. This step of the process produces a sponge-like collagen sheet. Next, the temperature inside the chamber was increased to 20°C and was held at this temperature for 16 hours. Finally, the pressure inside the chamber was reduced to 2.5 to 3 Pa (0.025 to 0.03 mbar) for 30 to 60 minutes.

After performing the above steps, a gel layer, such as that shown in Figure 3, was removed from each of the moulds. In comparison to a collagen sponge, such as that shown in Figure 2, the gel layer is noticeably different in physical appearance and its properties. The gel layer is transparent, pliable and tacky, unlike the collagen sponge.

### Water dissolution test

A gel layer obtained using the method described in the above Example was soaked in water for at least 12 hours. The soaked gel layer is shown in Figure 4.

No visible dissolution or disintegration of the gel layer occurred after soaking the gel layer in water for this period of time. The gel layer has absorbed water and is noticeably thicker than it was before soaking. The transparency of the gel layer after significant water absorption is also reduced.

### Example 2

Layered materials were prepared using the method described in Example 1, except that the amounts of glycerol and the 1% by weight aqueous dispersion of collagen used to prepare the materials were varied as shown in Table 1 below.

**Table 1**

| Sample | 1* | 2 | 3 | 4 | 5 | 6 | 7 |
|---|---|---|---|---|---|---|---|
| % wt glycerol | 0.75 | 1 | 2.5 | 5 | 7.5 | 10 | 20 |
| % wt of aqueous dispersion of collagen | 99.25 | 99 | 97.5 | 95 | 92.5 | 90 | 80 |

| | | | | | | | |
|---|---|---|---|---|---|---|---|
| *Reference Sample | | | | | | | |

Samples 2 to 7 above were transparent, tacky and non-porous gel layers. However, sample 1 was a porous and opaque material that was not tacky. Sample 1 had similar properties to a collagen sponge. This example shows that decreasing the amount of glycerol in the layered material can result in the formation of a collagen sponge-like product.

### Example 3

A gel layer was prepared as described in Example 1, except that gold nanoparticles having a diameter of 2 nm and methylene blue were added to the mixture of glycerol and collagen before it was placed in a mould and lyophilised. The gold nanoparticles and methylene blue photosensitiser that were used to prepare the gel layer are disclosed in WO 2008/015453.

The resulting gel layer had similar characteristics and handling properties to the gel layer of Example 1. This example shows that the gel layer of the invention is able to accommodate other agents without changing the handling properties of the material.

### Example 4

An area of a patient's skin was swabbed to determine the amount bacteria that were present. The amount of bacteria was measured using standard techniques (e.g. by culturing samples from the swab in a Petri dish and incubator, then counting the number of bacteria after incubation).

A gel layer of Example 1 was applied to the same area of skin. The gel layer was secured in position and was covered with a waterproof bandage covering to prevent absorption of moisture from the air. After the gel layer had been held in contact with the skin for 24 hours, it was removed and the uncovered area of skin was then swabbed. The amount of bacteria present on the skin that was covered by the gel layer was then determined.

It was found that the amount of bacteria present on the skin after the gel layer had been removed was substantially lower than the amount of bacteria that had been present before coverage with the gel layer. The gel layer may itself exhibit a surprising antimicrobial effect. It is believed that the bacteria present on the skin were absorbed into the gel layer matrix.

## Claims

1. A gel layer suitable for use as a skin dressing, wherein said gel layer does not have an open-pore structure and comprises a homogeneous mixture of glycerol and collagen, and the ratio of glycerol to collagen in the gel layer is from 50:1 to 1:1 by weight, wherein the gel layer is obtainable by lyophilising an aqueous mixture comprising 1 to 10% by weight of glycerol and 90 to 99% by weight of an aqueous dispersion of collagen, wherein the amount of collagen in the aqueous dispersion is from 0,3% to 5% by weight of the aqueous dispersion.

2. A gel layer according to claim 1, wherein the lyophilisation is performed for at least 15 hours.

3. A gel layer according to claim 1 or 2, wherein the amount of water in the gel layer is less than or equal to 1% by weight of the layer.

4. A gel layer according to any one of the preceding claims, wherein the collagen is porcine collagen.

5. A gel layer according to any one of the preceding claims, wherein the collagen is non-crosslinked atelopeptide collagen.

6. A gel layer according to any one of the preceding claims, wherein the ratio of glycerol to collagen in the gel layer is from 25:1 to 3:2 by weight.

7. A gel layer according to any one of the preceding claims, comprising an anti-ageing agent, an anti-wrinkle agent, an antioxidant, an anti-scarring agent, an antiseptic anti-acne agent or a combination thereof.

8. A gel layer according to any one of the preceding claims, comprising an antibiotic, an antibacterial agent, an antiviral agent, an antifungal agent, a growth factor, a corticosteroid, a prostaglandin, a cytokine, zinc ions, hyaluronic acid, fibronectin, a proteoglycan, an alginate or a combination thereof.

9. A gel layer according to claim 8, wherein the gel layer comprises gentamicin, and/or an alginate, and/or silver ions, and/or platelet derived growth factor (PDGF)

10. A gel layer according to any one of the preceding claims, wherein the gel layer comprises nanoparticles and photosensitiser(s).

11. A gel layer suitable for use as a skin dressing, wherein said layer comprises a homogeneous mixture of glycerol and non-crosslinked atelopeptide collagen, wherein the ratio of glycerol to collagen in the gel layer is from 50:1 to 1:1 by weight, and wherein the gel layer does not have an open-pore structure.

12. A wound dressing suitable for topical use, comprising a gel layer as defined in any one of claims 1 to 11, and a retaining layer for securing the dressing to skin or to a wound area.

13. A wound dressing according to claim 12, wherein the retaining layer is an outer layer of the dressing having at least one adhesive portion that extends beyond the gel layer.

14. A cosmetic method of improving the appearance of skin, which comprises applying a gel layer as defined in any one of claims 1 to 7 and 11 to the skin of a human or animal patient, and optionally wherein the gel layer comprises an anti-ageing agent, an anti-wrinkle agent or an antioxidant.

15. A gel layer as defined in any one of claims 1 to 11 for topical use in treating a wound, wherein the wound preferably is a bum.

## Patentansprüche

1. Gelschicht, die zur Verwendung als Hautverband geeignet ist, wobei die Gelschicht keine offenporige Struktur hat und eine homogene Mischung aus Glycerin und Collagen umfasst und das Gewichtsverhältnis von Glycerin zu Collagen in der Gelschicht 50:1 bis 1:1 beträgt, wobei die Gelschicht durch Lyophilisation einer wässrigen Mischung erhältlich ist, die 1 bis 10 Gew.-% Glycerin und 90 bis 99 Gew.-% einer wässrigen Collagendispersion umfasst, wobei die Menge an Collagen in der wässrigen Dispersion 0,3 bis 5 Gew.-% der wässrigen Dispersion beträgt.

2. Gelschicht nach Anspruch 1, wobei die Lyophilisation mindestens 15 Stunden lang durchgeführt wird.

3. Gelschicht nach Anspruch 1 oder 2, wobei die Menge an Wasser in der Gelschicht kleiner oder gleich 1 Gew.-% der Schicht ist.

4. Gelschicht nach einem der vorhergehenden Ansprüche, wobei das Collagen Schweinecollagen ist.

5. Gelschicht nach einem der vorhergehenden Ansprüche, wobei das Collagen nichtvernetztes Atelopeptidcollagen ist.

6. Gelschicht nach einem der vorhergehenden Ansprüche, wobei das Gewichtsverhältnis von Glycerin zu Collagen in der Gelschicht 25:1 bis 3:2 beträgt.

7. Gelschicht nach einem der vorhergehenden Ansprüche, mit einem Anti-Aging-Mittel, einem Antifaltenmittel, einem Antioxidationsmittel, einem Antinar-benbildungsmittel, einem antiseptischen Aknemittel oder einer Kombination daraus.

8. Gelschicht nach einem der vorhergehenden Ansprüche, mit einem Antibiotikum, einem antibakteriellen Mittel, einem antiviralen Mittel, einem Antimykotikum, einem Wachstumsfaktor, einem Corticosteroid, einem Prostaglandin, einem Cytokin, Zinkionen, Hyaluronsäure, Fibronectin, einem Proteoglycan, einem Alginat oder einer Kombination daraus.

9. Gelschicht nach Anspruch 8, wobei die Gelschicht Gentamicin und/oder ein Alginat und/oder Silberionen und/oder einen von Blutplättchen abgeleiteten Wachstumsfaktor (PDGF) umfasst.

10. Gelschicht nach einem der vorhergehenden Ansprüche, wobei die Gelschicht Nanopartikel und einen oder mehrere Photosensibilisatoren umfasst.

11. Gelschicht, die zur Verwendung als Hautverband geeignet ist, wobei die Schicht eine homogene Mischung aus Glycerin und nichtvernetztem Atelopeptidcollagen umfasst, wobei das Gewichtsverhältnis von Glycerin zu Collagen in der Gelschicht 50:1 bis 1:1 beträgt und wobei die Gelschicht keine offenporige Struktur hat.

12. Wundverband, der zur topischen Anwendung geeignet ist, mit einer Gelschicht nach einem der Ansprüche 1 bis 11 und einer Halteschicht zur Befestigung des Verbands an der Haut oder an einem Wundbereich.

13. Wundverband nach Anspruch 12, wobei die Halteschicht eine Außenschicht des Verbands mit wenigstens einem Haftabschnitt ist, der sich über die Gelschicht hinaus erstreckt.

14. Kosmetisches Verfahren zur Verbesserung des Erscheinungsbildes der Haut, bei dem eine Gelschicht nach einem der Ansprüche 1 bis 7 und 11 auf die Haut eines Human- oder Tierpatienten aufgebracht wird, und optional wobei die Gelschicht ein Anti-Aging-Mittel, ein Antifaltenmittel oder ein Antioxidationsmittel umfasst.

15. Gelschicht nach einem der Ansprüche 1 bis 11 zur topischen Anwendung bei der Behandlung einer Wunde, wobei die Wunde vorzugsweise eine Verbrennung ist.

## Revendications

1. Couche de gel adaptée à l'utilisation en tant que pansement cutané, la couche de gel ne présentant pas de structure à pores ouverts et comprenant un mélange homogène de glycérine et de collagène, et le rapport en poids entre la glycérine et le collagène dans la couche de gel étant de 50 : 1 à 1 : 1, la couche de gel pouvant être obtenue par lyophilisation d'un mélange aqueux comprenant de 1 à 10 % en poids de glycérine et 90 à 99 % en poids d'une dispersion aqueuse de collagène, la quantité de collagène dans la dispersion aqueuse étant de 0,3 % à 5 % en poids de la dispersion aqueuse.

2. Couche de gel selon la revendication 1, la lyophilisation étant réalisée pendant au moins 15 heures.

3. Couche de gel selon la revendication 1 ou 2, la quantité d'eau dans la couche de gel étant inférieure ou égale à 1 % en poids de la couche.

4. Couche de gel selon l'une des revendications précédentes, le collagène étant un collagène porcin.

5. Couche de gel selon l'une des revendications précédentes, le collagène étant un collagène atélopeptide non réticulé.

6. Couche de gel selon l'une des revendications précédentes, le rapport en poids ente la glycérine et le collagène dans la couche de gel étant de 25 : 1 à 3:2.

7. Couche de gel selon l'une des revendications précédentes, comprenant un agent anti-âge, un agent antirides, un antioxydant, un agent anti-cicatrices, un agent anti-acné antiseptique, ou une combinaison de ceux-ci.

8. Couche de gel selon l'une des revendications précédentes, comprenant un antibiotique, un agent antibactérien, un agent antiviral, un agent antimycosique, un facteur de croissance, un corticostéroïde, une prostaglandine, une cytokine, des ions de zinc, un acide hyaluronique, une fibronectine, un protéoglycane, un alginate ou une combinaison de ceux-ci.

9. Couche de gel selon la revendication 8, la couche de gel comprenant une gentamicine et/ou un alginate et/ou des ions d'argent et/ou un facteur de croissance dérivé des plaquettes (PDGF).

10. Couche de gel selon l'une des revendications précédentes, la couche de gel comprenant des nanoparticules et un ou plusieurs photosensibilisateur(s).

11. Couche de gel adaptée à l'utilisation en tant que pansement cutané, la couche comprenant un mélange homogène de glycérine et de collagène atélopeptide non-réticulé, le rapport en poids entre la glycérine et le collagène dans la couche de gel étant de 50 : 1 à 1 : 1, et la couche de gel ne présentant pas de structure à pores ouverts.

12. Pansement pour plaies adapté à l'utilisation topique, comprenant une couche de gel selon l'une des revendications 1 à 11 et une couche de retenue pour fixer le pansement sur la peau ou sur une zone de plaie.

13. Pansement pour plaies selon la revendication 12, la couche de retenue étant une couche extérieure du pansement qui présente au moins un tronçon adhésif qui s'étend au-delà de la couche de gel.

14. Procédé cosmétique pour l'amélioration de l'apparence de la peau, comprenant l'application d'une couche de gel selon l'une des revendications 1 à 7 et 11 sur la peau d'un patient humain ou animal, et dans lequel en option la couche de gel comprend un agent anti-âge, un agent antirides ou un antioxydant.

15. Couche de gel selon l'une des revendications 1 à 11 pour l'utilisation topique dans le traitement d'une plaie, la plaie étant de préférence une brûlure.
